# EUROPEAN PATENT SPECIFICATION

(11) **EP 1 646 349 B1**
(45) Date of publication and mention of the grant of the patent: **08.07.2009**
(21) Application number: 04732723.4
(22) Date of filing: 13.05.2004
(51) Int. Cl.: A61F 13/64

(54) **INCONTINENCE PADS**
INKONTINENZPRODUKT
TAMPONS D'INCONTINENCE

(30) Priority: 18.07.2003 IT CH20030012
(43) Date of publication of application: 19.04.2006
(73) Proprietor: D'Alcini, Franco, I-65124 Pescara (PE) (IT)
(72) Inventor: D'Alcini, Franco, I-65124 Pescara (PE) (IT)
(74) Representative: Baldi, Claudio
(86) International application number: PCT/IT2004/000269
(87) International publication number: WO 2005/007052

(56) References cited:
- WO-A-03/007863
- US-A- 5 445 628
- US-A1- 2001 034 511
- US-A1- 2001 034 512
- US-A1- 2002 045 881
- US-A1- 2002 193 776
- US-A1- 2003 069 558
- US-A1- 2003 120 253

## Description

The present patent application refers to an incontinence pad provided with two folding semi-belts.

As it is known, the function of Incontinence pads is to contain and absorb the organic products of evacuation (urine and faeces) in individuals to avoid uncontrolled diffusion on beds or clothes.

Traditional incontinence pads are composed of three layers of different materials: an external layer of filtering materials, a central thicker layer of absorbing fibres and an external layer of waterproof film.

Incontinence pads are basically shaped as a clepsydra and this shape allows them to fit as underpants once they are worn by the user.

When the traditional incontinence pad is worn by the user, the polyethylene lateral flaps located in the back and front section overlap on the user's hips and are held in place by means of adhesive bands, thus allowing to adjust the circumference of the incontinence pad to the user's physical characteristics.

The aforementioned overlapping of the flaps on the user's hips is the main drawback of the traditional technology.

The presence of the overlapped flaps on the user's hips completely prevents perspiration in that area.

If we consider that the overlapped flaps of incontinence pads are made with anti-perspirant waterproof material, it appears evident that, in addition to cause excessive sweating, traditional incontinence pads may cause severe cutaneous irritation to the user.

The purpose of the present invention is to provide an incontinence pad that, in spite of having a general structure similar to the traditional one, features an innovative fastening system without the disadvantageous overlapping of the lateral waterproof flaps on the user's hips.

The shape of the incontinence pad of the invention is basically the same as traditional incontinence pads, with the only significant difference that it does not feature the protruding lateral flaps (on the back and front section) used by the traditional technology to hold the incontinence pad in place on the user's hips.

While on one hand the absence of the lateral flaps overlapped on the user's hips improves perspiration, on the other hand it prevents from using the traditional fastening system with lateral adhesive bands.

For this reason, the incontinence pad of the invention is provided with a closing belt on the back section. When the closing belt is not used, the right and left ends of the belt (semi-belts) are folded one on top of the other.

When the incontinence pad is worn by the user - that is to say when the incontinence pad is used as underpants - the two semi-belts are stretched out by pulling them slightly and surround the user's waist from the back to the front, with one semi-belt on the left side and one semi-belt on the right side.

In view of the above, the user's hips are no longer covered by a double layer of waterproof anti-perspirant material. Instead they are covered by the semi-belts, which are made of a normally perspiring material.

The internal side of the free end of one semi-belt is provided with an insert of adhesive material that allows to hold the belt in place on the front section of the incontinence pad when the same is worn by the user.

The specific fastening point of the free end of each semi-belt can be established according to the size of the user's waist, thus ensuring perfect adjustment of the incontinence pad/underpants to the user's body.

In particular, the user can try different fastening positions, since each semi-belt can be attached and detached several times on the front section of the incontinence pad.

US patent No. 2002/0193776 A1 describes an absorbent article comprising a front portion, a back portion and a crotch portion therebetween to be folded in such a way that the article assumes a pant-like shape, leaving the hips uncovered; said absorbent article being also provided with a belt attached to the back portion and long enough to surround the waist and be fixed on the outside of the front portion. More precisely said belt is not a single piece but is composed of first and second belt halves the ends of which are fastened, during packaging, to the aforementioned back portion of the absorbent article, on the permeable topsheet which is destined to be in contact with the user's skin.

Also during packaging said two halves remain substantially flat along their entire length when brought against said topsheet on top of each other. US patent No. 2001/0034512 A1 describes an absorbent article such as an incontinence guard basically identical to the article illustrated in US patent No. 2002/193776 A1 except for the fact that during packaging the two halves are folded in accordion-like fashion and each forms an accordion-like folded package which is arranged in a pocket at each side edge of the rear portion of the absorbent article.

Also in the item according to this second device the two belt halves are fastened on the inside of the permeable sheet which remains on the internal side of the pant when the article is used.

A further purpose of the invention according to the present application is to provide a new method of folding and fastening said belt to the back portion of the incontinence pad in such a way that the two semi-belts are not allowed to move freely thus not impairing packaging and handling operations.

As illustrated in details in the description below, special attention has been paid to the way the semi-belts are folded and fastened on the back of the incontinence pad.

When the incontinence pad is not used, the two semi-belts must be positioned in such a way as not to preclude normal packaging of incontinence pads.

At the same time, the two semi-belts must be folded and fastened in such a way as to remain one against the other and be stretched out completely by the user with his/her hands.

In particular, the compressed steady position of the semi-belt is extremely useful during packaging and handling. Should the semi-belts be allowed to move freely, packaging and handling operations would be severely impaired.

The advantages of the incontinence pad of the invention appear evident from the aforementioned generic description: it favours perspiration on the user's hips, has excellent wearability (meaning the possibility of adjusting to the specific requirements of different users), and reduces the quantity of materials and glue used compared to traditional incontinence pads, thanks to the elimination of lateral flaps.

It is worthless saying that the reduced consumption of these parts reduces the production cost of the incontinence pad of the invention and also the environmental impact related to the disposal of throwaway incontinence pads.

For major clarity the description of the invention continues with reference to the enclosed drawings, which are intended for purposes of Illustration only and not in a limiting sense, whereby:
- Figs. 1 to 4 are perspective views of the incontinence pad of the invention in operational condition in the different steps of practical application on the user;
- Figs. 5 to 7 show the different steps of the folding process for the belt of the incontinence pad;
- Fig. 8 is a plan view of a section of the belt.

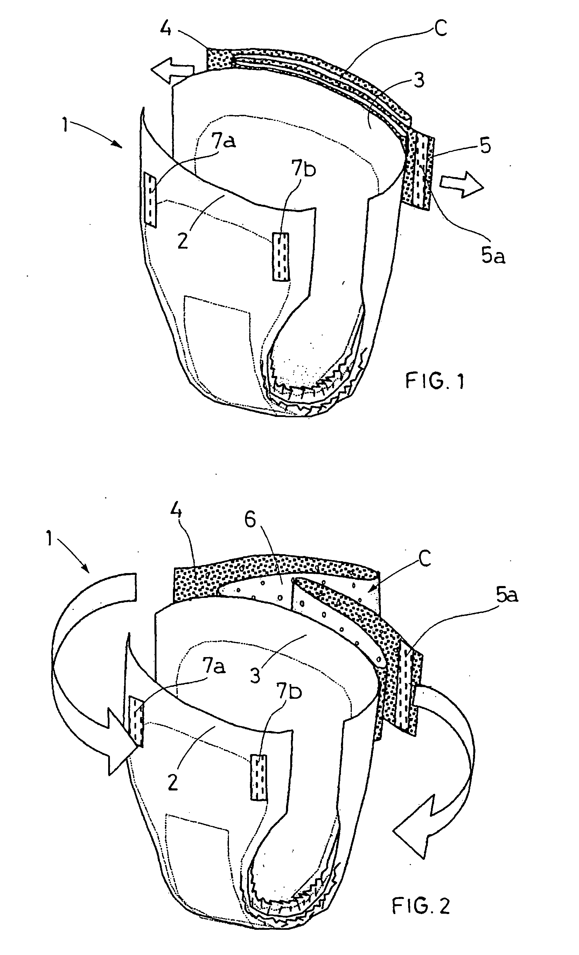

With reference to Figs. 1 to 4, the incontinence pad of the invention (1), which basically has the traditional "clepsydra" shape, is characterised by the fact that the length of the front transversal border (2) and back transversal border (3) only allows to cover the user's back and stomach - and not the hips - when the incontinence pad is worn.

As mentioned above, the presence of the two free lateral areas provides the incontinence pad (1) with a level of comfort and hygiene that is unknown in traditional incontinence pads.

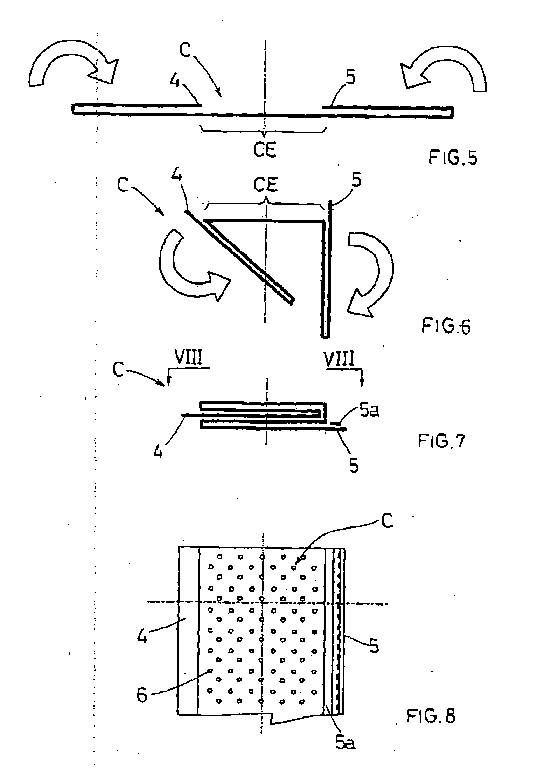

A belt (C) is fastened on the outside of the back transversal border (3), whose right and left ends (4, 5) - or semi-belts - are folded one against the other according to the preferred sequence shown in Figs. 5 to 7.

It must be pointed out that the belt (C) is fastened on the outside of the back transversal border (3), on the sheet which remains on the external side of the pant when the Incontinence pad is used.

As mentioned earlier, the function of the semi-belts (4, 5) is to connect the front border (2) and back border (3) of the incontinence pad (1) from both sides, thus surrounding the user's hips without preventing perspiration.

The semi-belts (4, 5) must be provided with perspirant properties and can be made of different suitable materials, such as "non-woven" transversally non-elastic fabric of single-layer or double-layer type, or "elasticised non woven" fabric or composite material (partially elastic and partially rigid).

To compress and stabilise the two semi-belts (4, 5) one against the other in folded position, the two semi-belts (4, 5) are subjected to compression, preferably by means of lamination.

In order to improve the efficacy of compression on the semi-belts (4, 5) the surface of one of the lamination rolls can be provided with a dense series of small points to perforate the layers of the folding section, thus creating a sort of punctiform union between the different layers.

Fig. 8 shows a section of the semi-belt after being subjected to this type of operation, in which number (6) shows the perforations created by the punctiform union.

Alternatively, the union between the two semi-belts in contact position can be obtained by means of ultrasounds.

In both cases, however; the user can separate the different sections of the folded part by simply pulling the free ends of the two semi-belts (4, 5) towards the front of the incontinence pad (1), as shown in Fig. 2.

Once the two semi-belts (4, 5) have been completely unfolded, the user simply needs to fasten them on the front of the incontinence pad (1).

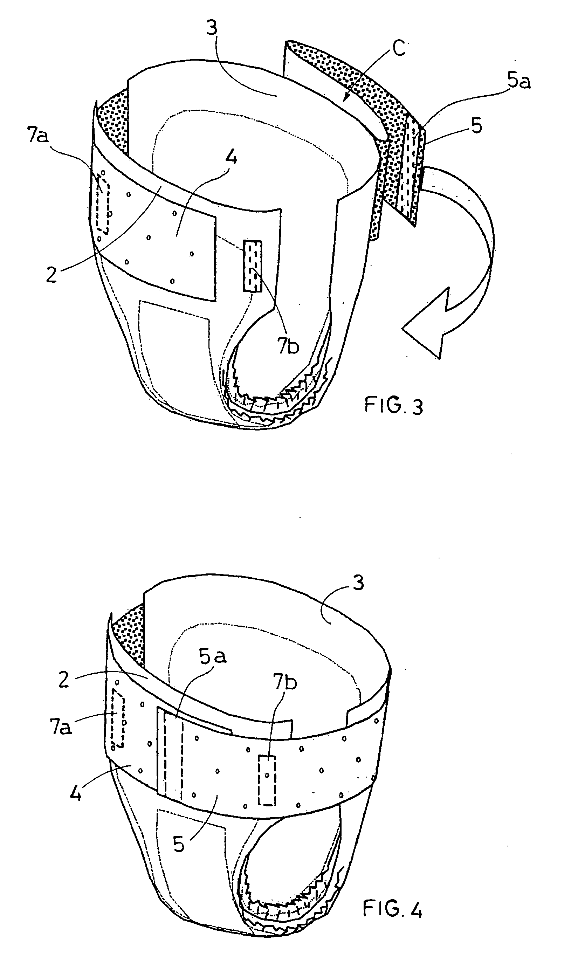

To that end, the two sides of the front section of the incontinence pad (1) are provided with Velcro (7a, 7b) or other adhesive materials in order for the Internal sides of the semi-belts (4, 5) to adhere, according to the sequence shown in Figs. 3 and 4.

With reference to the same figures, the right semi-belt (4) is fastened against the front adhesive insert (7a) and the left semi-beft (5) is fastened against the corresponding adhesive insert (7b).

The length of the semi-belts (4, 5) is such that the semi-belts (4, 5) can overlap approximately on the user's belly-button.

To ensure the stable fastening of the two semi-belts (4, 5), the free end of the left semi-belt (5) is internally provided with an adhesive insert (5a) that allows for fastening it on the external side of the right semi-belt (4), as shown in Fig. 4.

In view of the fact that the front adhesive inserts (7a, 7b) are capable of adhering anywhere on the internal side of the two semi-belts (4, 5) it appears evident that the two semi-belts (4, 5) are finally fastened against the adhesive inserts after adjusting their tension according to the user's waist.

The user can therefore try different fastening positions by attaching and detaching the two semi-belts (4, 5) to the fastening inserts (7a, 7b) several times before finding the best position for each user.

## Claims

1. Incontinence pad, of the type comprising a front transversal border (2) and a back transversal border (3) designed to assume a pant-like shape leaving the hips uncovered when the pad is worn and a belt (C) fastened to the back border (3) and long enough to surround the waist and be fixed on the outside of the front border (2), it being provided that a central section (CE) of the belt (C) is fastened on the outside of the back border (3) and features right and left ends (4, 5) designed to surround and be fastened on the outside of the front border (2) when the pad is used; incontinence pad **characterised in that**:
- said right and left ends (4, 5) of the belt (C) are folded and compressed one against the other and both against the external side of said central section (CE) of the belt (C), when the pad is not used;
- the folded right and left ends (4, 5) of the belt (C) are also compressed by means of lamination, it being provided that the surface of at least one of the lamination rolls features a dense regular series of small points capable of perforating the overlapped layers of the ends (4, 5).

2. Incontinence pad as defined in claim 1, **characterised by** the fact that the free end of the left semi-belt (5) is internally provided with an adhesive insert (5a) that allows for fastening it on the external side of the right semi-belt (4) after overlapping the semi-belts on the incontinence pad (1).

3. Incontinence pad as defined in one or more of the preceding claims, **characterised by** the fact that the belt (C) is made of single-layer transversally non-elastic material, consisting in "non woven" co-extruded fibres.

4. Incontinence pad as defined in one or more of the preceding claims, **characterised by** the fact that the belt (C) is made of double-layer transversally non-elastic material, consisting in "non woven" co-extruded fibres.

5. Incontinence pad as defined in one or more of the preceding claims, **characterised by** the fact that the belt (C) is made of composite material (partially elastic and partially rigid) of co-extruded or laminated fibres.

## Patentansprüche

1. Windel für Inkontinenz-Betroffene mit einer vorderen Querkante (2) und einer hinteren Querkante (3), die dazu dienen, die Windel bei Gebrauch in eine Unterhose zu verwandeln, die die Hüfte des Benutzers frei lässt, sowie mit einem Gürtel (C), der an der oberen Kante (3) befestigt wird und ausreichend lang ist, um um die Taille gewickelt und auf der Außenseite der vorderen Kante (2) befestigt zu werden, wobei der mittlere Bereich (CE) des Gürtels (C) auf der Außenseite der rückwärtigen Kante (3) befestigt ist und ein rechtes bzw. ein linkes Ende (4 und 5) aufweist, die dazu dienen, bei Gebrauch um die Taille gewickelt und an der Außenseite der vorderen Kante (2) befestigt zu werden, **dadurch gekennzeichnet, dass**:
- das rechte und das linke Ende (4 und 5) des Gürtels (C) vor Gebrauch blasebalgartig zusammengefaltet und aneinanderliegend an die Außenseite des zuvor genannten mittleren Bereichs (CE) des Gürtels (C) gepresst sind;
- das zusammengefaltete rechte und linke Ende (4 und 5) des Gürtels (C) einem Walzenpressverfahren unterzogen werden, wobei vorgesehen ist, dass mindestens eine der Walzrollen auf ihrer Oberfläche eine Reihe von kleinen, regelmäßig angeordneten Dornen aufweist, die in der Lage sind, alle übereinanderliegenden Schichten der Enden (4 und 5) zu durchbohren.

2. Windel gemäß Anspruch 1, **dadurch gekennzeichnet, dass** das freie Ende der linken Gürtelhälfte (5) inwendig einen Klebeeinsatz (5a) aufweist, der auf der Außenseite der rechten Gürtelhälfte (4) haftet, wenn diese auf der Vorderseite der Windel (1) übereinandergelegt werden.

3. Windel gemäß einem oder mehreren der vorstehenden Ansprüche, **dadurch gekennzeichnet, dass** der Gürtel (C) aus einem in Querrichtung unelastischen, einschichtigen Material gefertigt ist, das aus "non woven"-Co-Extrusionsfasern besteht.

4. Windel gemäß einem oder mehreren der vorstehenden Ansprüche, **dadurch gekennzeichnet, dass** der Gürtel (C) aus einem in Querrichtung unelastischen, doppelschichtigen Material gefertigt ist, das aus "non woven"-Co-Extrusionsfasern besteht.

5. Windel gemäß einem oder mehreren der vorstehenden Ansprüche, **dadurch gekennzeichnet, dass** der Gürtel (C) aus einem (teils elastischen und teils starren) Kompositmaterial gefertigt ist, das aus Co-Extrusionsfasern oder Pressspan besteht.

## Revendications

1. Couche-culotte pour incontinents, du type comprenant un bord transversal antérieur (2) et un bord postérieur (3) aptes à assumer, lors de l'usage, la conformation d'une culotte qui laisse découvertes les hanches de l'usager, ainsi qu'une ceinture (C), fixée sur le bord postérieur (3), ayant une longueur suffisante à l'enrouler et à la fixer à l'externe du bord antérieur (2), dite ceinture (C) étant fixée, sur un segment de sa partie centrale (CE), à l'externe du bord postérieur (3) et présentant deux bouts libres de droite et de gauche (4 et 5), aptes à être enroulés et fixés à l'externe du bord antérieur (2) lors de l'usage ; couche-culotte **caractérisée en ce que** :
- les dits bouts de droite et de gauche (4 et 5), avant l'usage, sont repliés en accordéon et compactés par écrasement l'un contre l'autre et les deux contre la paroi externe du dit segment central (CE) de la ceinture (C) avant usage ;
- les dits bouts de droite et de gauche (4 et 5) ont été soumis, après avoir été repliés en accordéon à l'externe du segment central (CE), à un processus de compactage par laminage, en prévoyant qu'au moins l'un des rouleaux de laminage présente sur sa surface une série régulière de petites pointes aptes à transpercer toutes les couches superposées des bouts (4 et 5).

2. Couche-culotte selon la revendication 1, **caractérisée en ce que** le bout de gauche (5) présente, sur l'interne de son extrémité libre, un insert adhésif (5a) en mesure d'adhérer contre la surface externe du bout de droite (4) lorsque leur superposition sera effectuée sur la partie avant de la couche-culotte (1) même.

3. Couche-culotte selon l'une ou plusieurs des revendications précédentes, **caractérisée en ce que** la dite ceinture (C) est réalisée dans un matériau du type monocouche non élastique dans le sens transversal, consistant de fibres coextrudées du type "non-tissé".

4. Couche-culotte selon l'une ou plusieurs des revendications précédentes, **caractérisée en ce que** la dite ceinture (C) est réalisée dans un matériau du type bicouche non élastique dans le sens transversal, consistant de fibres coextrudées du type "non-tissé".

5. Couche-culotte selon l'une ou plusieurs des revendications précédentes, **caractérisée en ce que** la dite ceinture (C) est réalisée dans un matériau composite (en partie élastique et en partie rigide) de fibres coextrudées ou laminées.
